(19) Europäisches Patentamt

European Patent Office

Office européen des brevets

(11) **EP 0 994 346 B1**

(12) **EUROPÄISCHE PATENTSCHRIFT**

(45) Veröffentlichungstag und Bekanntmachung des Hinweises auf die Patenterteilung:
**19.01.2005 Patentblatt 2005/03**

(51) Int Cl.7: **G01N 27/417**, B01D 53/86, B01D 53/30

(21) Anmeldenummer: **99119164.4**

(22) Anmeldetag: **06.10.1999**

(54) **Messsonde für die Detektion der Momentankonzentrationen mehrerer Gasbestandteile eines Gases**

Sensor for the detection of actual concentrations of several gas compounds in a gas

Sonde pour la détection des concentrations actuelles de plusieurs composants gazeux dans un gaz

(84) Benannte Vertragsstaaten:
**AT BE CH CY DE DK ES FI FR GB GR IE IT LI LU MC NL PT SE**

(30) Priorität: **09.10.1998 DE 19846487**

(43) Veröffentlichungstag der Anmeldung:
**19.04.2000 Patentblatt 2000/16**

(73) Patentinhaber: **BASF AKTIENGESELLSCHAFT 67056 Ludwigshafen (DE)**

(72) Erfinder:
• **Odermatt, Peter**
  **67098 Bad Dürkheim (DE)**
• **Spiegel, Andreas**
  **67061 Ludwigshafen (DE)**
• **Dittrich, Jürgen**
  **76532 Baden-Baden (DE)**

(74) Vertreter: **Isenbruck, Günter, Dr. et al Isenbruck, Bösl, Hörschler, Wichmann, Huhn, Patentanwälte Theodor-Heuss-Anlage 12 68165 Mannheim (DE)**

(56) Entgegenhaltungen:
**EP-A- 0 239 106        EP-A- 0 496 003
EP-A- 0 820 799        WO-A-97/42495
US-A- 5 540 047**

**Beschreibung**

Die Erfindung betrifft eine Meßsonde für die Detektion der Momentankonzentrationen mehrerer Gasbestandteile eines Gases, insbesondere zur Abgasmessung, und Verbrennungsabgase aus Verbrennungsanlagen für fossile oder biologische Brennstoffe bzw. Abfälle oder von Verbrennungskraftmaschinen. Beispielsweise Automotoren nach dem Dieselprinzip enthalten Schadstoffe, die eine Umweltbelastung darstellen. Besonders die Stickstoffoxide (Stickoxide) als toxische und umweltgefährdende Substanzen stehen im Mittelpunkt des öffentlichen Interesses und sollen möglichst vollständig aus den Verbrennungsabgasen entfernt werden, um eine Umweltbelastung zu vermeiden. Beispiele solcher Stickoxide sind NO, $NO_2$, $NO_3$, $N_2O_3$, $N_2O_4$, und $N_2O_5$. Diese sind aus der Lehrbuchliteratur bekannt, von besonderer Bedeutung ist jedoch NO und $NO_2$.

Ein Dieselabgas kann gemäß der Literaturstelle Kraftfahrtechnisches Taschenbuch, Robert Bosch GmbH, 1991, S. 513, folgende Zusammensetzungen aufweisen:

| | |
|---|---|
| $NO_x$ | 50 - 2500 ppm, |
| KW | 50 - 500 ppm $C_1$ |
| CO | 100 - 2000 ppm |
| Ruß | 20 - 200 mg/m$^3$ |
| $H_2O$ (Dampf) | 2 - 11 Vol. % |
| $O_2$ | 2 - 18 Vol%, |
| $CO_2$ | 0 - 16 Vol%, |
| $N_2$ | Rest |

KW bedeutet Kohlenwasserstoffe, $H_2O$ liegt in Dampfform vor, die Konzentrationen beziehen sich auf Volumina. Die Temperatur beträgt dabei 100 - 750 °C, der nicht angegebene Druck wird mit etwa 1 bis 1,05 bar angenommen. Zusätzlich kann $SO_2$ vorhanden sein.

Die Stickoxide $NO_x$ werden häufig mit Ammoniak oder Ammoniak abgebenden Stoffen wie Harnstoff, Ammoniumcarbonat, Ammoniumhydrogencarbonat, Ammoniumcyanat und anderen reduziert. Der Harnstoff kann dabei beispielsweise in einer 30%igen wäßrigen Lösung, $NH_3$ als Gas, eingespeist werden. Die Reduktion läuft dabei nach folgenden Umsetzungsgleichungen ab:

$$4\,NO + 4\,NH_3 + O_2 \rightarrow 4\,N_2 + 6\,H_2O$$

$$NO + NO_2 + 2NH_3 \rightarrow 2\,N_2 + 3\,H_2O$$

$$2\,NO_2 + 4\,NH_3 + O_2 \rightarrow 3\,N_2 + 6\,H_2O$$

Um die Stickoxide möglichst vollständig umzusetzen, gibt man zweckmäßig einen gleichmolaren oder höheren Anteil Ammoniak hinzu. Die Molgewichte sind für $NH_3$ = 17, für NO2 46, für $NO_3$ 62 und für $(N_2O_5)/2$ 54. Für eine stöchiometrische Umsetzung braucht man den ca. dreifachen Gewichtsanteil bzw. einfachen Volumenanteil Ammoniak, bezogen auf $NO_x$. Nimmt man bei der oben angegebenen Zusammensetzung mit durchschnittlich 2000 ppm $NO_x$ einen 3%igen Überschuß Ammoniak an, so verbleiben 60 ppm Ammoniak im Abgas. Sollen diese nicht überschritten werden, so muß die Messung 60 ppm mit einer Sicherheit von 10 ppm noch sicher erfassen können. Die Messung muß noch sicher funktionieren bei Abgastemperaturen von 100 bis 750°C. Ferner darf sie nicht gestört werden durch Ruß oder Stäube von Ammoniumsalzen und der Sensor darf nicht durch Korrosion durch z.B. Schwefeloxide beeinflußt werden.

Bei falschen Messungen läuft man Gefahr, eines der Umweitgifte Ammoniak oder Stickoxid in größerem Überschuß in den Abgasen zu haben. Beides ist in der Umwelt sehr unerwünscht Man bemüht sich, die Konzentration der Stickoxide unter den gesetzlich zulässigen Wert zu bringen und dabei kein überschüssiges Ammoniak hinzuzufügen.

In der DE-A-3 721 572 wird zur selektiven katalytischen Reduktion von Stickstoffoxiden ($NO_x$) in den Abgasen eines Motors die $NO_x$-Konzentration gemessen und in Abhängigkeit von der festgestellten $NO_x$-Konzentration zwecks Umsetzung an einem Katalysator $NH_3$ zugeführt. Die Regelung der $NH_3$-Menge erfolgt dabei zu mindestens 75% mittels der Motorlastdaten, der verbleibende Teil wird in Abhängigkeit von der in den Abgasen gemessenen $NO_x$-Konzentration geregelt. Man erhofft sich davon, die bislang bekannten trägen Regelungen der $NH_3$-Zufuhr anhand einer Messung der $NO_x$-Konzentration nach dem Katalysator bei instationärer Betriebsweise, bei der sich Leistung und Drehzahl der Motors und damit die $NO_x$-Konzentration in der Rohemission des Motors rasch verändern, zu verbessern. Diese Reaktionszeit wird mit etwa 1 Minute angegeben, während der bei geänderter Abgasmenge und Abgaszusammensetzung die Ammoniakzugabe noch konstant bleibt, weil die träge Meßsonde die Änderung noch nicht festgestellt hat.

Aus der EP-B-0 447 537 ist weiterhin bekannt, daß zum Betrieb eines Oxidationskatalysators der $NH_3$-Anteil möglichst gering sein muß, um die katalytische Wirkung zur Zerstörung von Dioxinen zu erhalten.

In der Fachzeitschrift "Sensors and Actuators", B4, 1991, Seite 530, wird eine Ansprechzeit von 30 s angegeben. Es handelt sich hier um das im Handel befindliche SOLIDOX-$NH_3$-System. Auch hier ist die Meßzeit noch zu lang, um eine instationäre Betriebsweise ausreichend zu regeln.

Aus der US-A-2,310,472 ist bekannt, Autoabgase dadurch zu analysieren, daß diese katalytisch verbrannt und die Temperaturerhöhung als Widerstandserhöhung des Verbrennungskatalysators gemessen wird. Dies erfolgt mittels einer Wheatstoneschen Brücke. Als Katalysator dient ein mit Ceroxid ummantelter Platin-Draht oder ein Filament aus katalytischem Material wie Platin. Die Zugabe von Ammoniak für die katalytische $NO_x$-Reduktion des Autoabgases ist nicht erwähnt.

Aus der US-A-2,583,930 ist bekannt, brennbare Gase oder Dämpfe dadurch zu analysieren, daß sie katalytisch verbrannt werden. Als Katalysator ist ein Platindraht oder ein Platin-Rodium-Draht vorgeschlagen, dessen Widerstandsänderung gemessen wird, wobei wiederum eine Wheatstonesche Brücke verwendet wird. Der besondere Vorteil ist hier die Vermeidung einer Drift.

In der EP-A-0 591 240 ist ein Sensor zur Messung von Ammoniak offenbart Der Sensor ist durch eine auf einer Oxidoberfläche angebrachte dünne Schicht von Platin oder Palladium empfindlich auf einen Gasbestandteil. Katalysiert wird z.B. eine Verbrennungsreaktion, und das primäre Signal ist der elektrische Widerstand eines Halbleiters. Durch mindestens zwei Sätze von Elektroden, die in unterschiedlichen Abständen auf dem Halbleiter angebracht sind, wird eine Funktionsverschlechterung erkannt.

Aus der US-A-3,586,486 ist die Analyse eines Autoabgases bekannt, bei der als Katalysator für die Verbrennung Platin als Platinschwarz-Film in einer Dikke von 0,0508 mm (0,002 Zoll) eingesetzt wird. Der Widerstand des katalytischen Widerstandselements hängt von der Temperatur ab, die Messung erfolgt mit einer Wheatstoneschen Brücke.

Daß der vorbeschriebene Sensor nicht befriedigend arbeitet, stellt die US-A-4,197,089 fest, die als Sensor einen $WO_3$-Film vorschlägt. Dieser wird für $NH_3$ durch eine kleine Menge eines Platin-Katalysators sensibilisiert. Der Platin-Katalysator liegt als dünne Schicht unter dem $WO_3$-Film. Die Widerstandsänderung wird mittels einer Wheatstoneschen Brücke gemessen, wobei der Widerstand des $WO_3$-Films durch das Reduktionsmittel $H_2S$ oder $NH_3$ sinkt.

Aus der DE-A-4 117 143 ist bekannt, den Anteil von $NH_3$ in Autoabgasen aus Kraftfahrzeugdieselmotoren zu analysieren. $NH_3$ wird katalytisch oxidiert, die durch die Wärmetönung bewirkte Temperaturerhöhung des Gases wird als Maß für die $NH_3$-Konzentration genommen. Der Katalysator wird innerhalb des Wabenkanals plaziert, alternativ kann auch ein Teilgasstrom entnommen werden. Weiterhin wird eine stöchiometrische Zudosierung von $NH_3$ bei konstantem Vollastbetrieb offenbart, im übrigen ist aber die getaktete überstöchiometrische Zugabe Gegenstand dieser Druckschrift.

In der DE-C-3 543 818 ist beschrieben, wie auf dieser Basis eine elektrochemische $ZrO_2$-Zelle zur Sauerstoffkonzentrationsmessung in Gasen funktioniert. Ein anderes, ebenfalls bekanntes Verfahren zur Messung der Sauerstoffkonzentration ist aus einem Merkblatt der Firma Dittrich Elektronik, Bahnhofstraße 67 in 76532 Baden-Baden bekannt, welches zusammen mit der Sauerstoffmeßzelle im Handel erhältlich ist.

Aus der WO 97/42495 ist eine elektrochemische Festkörperzelle bekannt, die zur Messung von Komponenten einer Gasmischung dient. Der Sensor umfasst eine Halbleiterelektrode mit zwei sich gegenüberliegenden Oberflächen, umfassend ein Halbleitermaterial, das in Kontakt mit der jeweiligen Komponente eine Änderung eines spezifischen Widerstandes erfährt. Ferner enthält der Sensor eine Metallelektrode, mit zwei sich gegenüberliegenden Oberflächen, und einen Elektrolyten, der mit der zweiten Oberfläche der Halbleiterelektrode und der zweiten Oberfläche der Metallelektrode in Kontakt steht. Dabei kontaktieren beide ersten Oberflächen die Gasmischung.

Die Langsamkeit der Sensoren ist immer wieder ein Problem, da zu wenig Meßdaten bzw. eine zu lange Meßzeit die Regelung der instationären Betriebsweise erschweren.

Wesentlich kürzere Reaktionszeiten erhält man mit einer Meßsonde, mit der gleichzeitig die Ermittlung der Momentankonzentration von Sauerstoff, Ammoniak und Stickoxid in Verbrennungsabgasen erfolgt. Dabei sind drei unterschiedliche Bereiche vorgesehen, von welchen in einem davon die Sauerstoff-Konzentration erfaßt und in den beiden anderen Bereichen vorzugsweise auf Basis des Sauerstoffverbrauchs bei der teilselektiven Umsetzung von Ammoniak bzw. Stickoxiden und Ammoniak mit Sauerstoff die Konzentration von $NH_3$ und $NO$ bestimmt wird. Auch durch eine Temperaturerhöhung, die durch eine exotherme Amoniak - bzw. Ammoniakkonzentrationen von besonderer Bedeutung. So soll die in den Verbrennungsabgasen enthaltene Ammoniakkonzentration von ca. 60 ppm mit einer Genauigkeit von 10 ppm kontinuierlich gemessen werden, damit auf der Basis des Meßergebnisses die Dosierung des Ammoniaks so erfolgt, daß keine nennenswerte Umweltschädigung hervorgerufen wird.

Neben der Messung von $O_2$, $NH_3$ und $NO$ kann es vorteilhaft sein, die CO-Konzentration in einem vierten Bereich zu erfassen. Als Katalysator kommt hier beispielsweise Gold in Betracht.

Besonders geeignet ist eine Meßsonde, deren erster Bereich zur Erfassung des Partialdrucks von $O_2$ ausgebildet ist, deren zweiter Bereich einen Reaktionskatalysator zumindest zur teilselektiven Umsetzung von $NH_3$ mit $O_2$ aufweist und deren dritter Bereich einen Reaktionskatalysator zumindest zur teilselektiven Umsetzung von $NO$ und $NH_3$ mit $O_2$ aufweist.

Auch durch Messung der Verbrennungswärme an einem Reaktionskatalysator, die beispielsweise eine Erhöhung des elektrischen Widerstands zur Folge hat, kann die Temperaturerhöhung zur Ammoniakkonzentration in Beziehung gesetzt werden. Es ist davon auszugehen, daß die geeigneten Reaktionskatalysatoren elektrische Leiter sind, deren elektrischer Widerstand mit steigender Temperatur gut meßbar zunimmt.

Die Anordnung der Bereiche auf einem gemeinsamen Träger hat den Vorteil, daß für alle drei Bereiche dieselben Bedingungen vorliegen. Dies ist insbesondere dann von Bedeutung, wenn der Träger sauerstoffleitend ausgebildet ist und zusammen mit den Bereichen gasgefüllte Kammern begrenzt, in welchen ein Referenzdruck von Sauerstoff eingestellt ist. Die Verwendung eines gemeinsamen Trägers stellt in diesem Fall die Gleichheit der Sauerstoffkonzentrationen her. Die Bedingungen können sich nämlich längs des Gasstromes ändern. So ist am Anfang des Katalysators, wo noch wenig Umsetzung stattgefunden hat, der Gehalt an $O_2$, $NH_3$ und NO relativ hoch, während die Gehalte gegen Ende des Katalysators, was auch beabsichtigt ist, stark abnehmen.

Gemäß der Erfindung können die Gehalte eng beieinander an derselben Stelle gemessen werden.

Um eine Versottung der Meßsonde zu vermeiden und die Dissoziierung des Sauerstoffmoleküls zu begünstigen, kann eine Heizvorrichtung vorgesehen sein. Vorteilhafterweise ist die Heizvorrichtung derart dimensioniert, daß in den ersten bis dritten Bereichen eine Temperatur von 500-900°C erreicht wird, vorzugsweise 700°C.

Als Meßsonde eignet sich insbesondere ein Aufbau mit einem Träger aus einem Material, in welchem $O_2$ in Form von Ionen transportiert werden kann. Der Träger ist auf einer Seite mit einer Referenzschicht aus einem Referenzmaterial verbunden und weist auf der anderen Seite ebenfalls eine Schicht aus dem Referenzmaterial auf, in welcher Kammern angeordnet sind, wobei die Kammern durch Scheiben aus einem Material, in welchem $O_2$ in Form von Ionen transportiert werden kann, abgedeckt und insgesamt gasdicht verschlossen sind, wobei auf den Scheiben Reaktionskatalysatoren angeordnet sind. Das Referenzmaterial der Referenzschicht bewirkt unter Stromeinwirkung den Transport von Sauerstoff in Form von Ionen durch den Träger in die Kammern und stellt so eine Bezugsgröße für das Partialdruckgefälle des Sauerstoffs in der Kammer gegenüber dem des zu messenden Gasstroms dar. In allen drei Bereichen wird ein $O_2$-Partialdruck gemessen.

Vorteilhafterweise ist die Meßsonde mit Mitteln zur Erfassung der Spannung zwischen den Reaktionskatalysatoren und der Referenzschicht versehen.

Zur Befestigung der Scheiben auf dem Träger ist ein Glaskleber vorteilhaft. Der Glaskleber ist nicht sauerstoffleitend, selbst bei den hohen Betriebstemperaturen, und dichtet die Kammern ab.

Ein weiterer Gegenstand der vorliegenden Erfindung ist der Einsatz der erfindungsgemäßen Meßsonde in einem Katalysatorsystem, in welchem zur Reduktion von Stickoxiden in Verbrennungsgasen $NH_3$ zugeführt wird, wobei die Meßsonde zumindest am Katalysatorausgang und/oder im Katalysator nach der Stelle der $NH_3$-Einspeisung angeordnet. ist. Vorteilhafterweise kann zusätzlich noch unmittelbar vor dem Katalysator und/oder vor einer Stelle der $NH_3$-Einspeisung eine Meßsonde angebracht sein. Im Gegensatz zu den bislang bekannten Katalysatorsystemen wird durch die punktförmige, reaktionsschnelle Messung der Stickoxide in den Verbrennungsgasen eine fein dosierte $NH_3$-Einspeisung möglich, so daß kein Überschuß von $NH_3$ aus dem Katalysatorsystem austritt.

Dank der erfindungsgemäßen Meßsonde läßt sich ein weiteres Katalysatorsystem realisieren, welches nach einem DeNO$_x$-Katalysator noch zusätzlich einen Dioxinumsetzenden Katalysatorteil enthält. Voraussetzung für die Umsetzung von Dioxin in dem Katalysator ist, daß die $NH_3$-Konzentration unterhalb eines gewissen Grenzwerts bleibt.

Vorteilhafterweise wird die $NH_3$-Einspeisung aufgrund der Meßergebnisse der erfindungsgemäßen Meßsonde geregelt.

In der Zeichnung ist ein Ausführungsbeispiel einer erfindungsgemäßen Meßsonde dargestellt. Es zeigt die:

Fig. 1    eine Meßsonde in einem Querschnitt, die

Fig. 2    die Meßsonde aus Fig. 1 in vergrößerter Darstellung und die

Fig. 3    ein Katalysatorsystem mit der erfindungsgemäßen Meßsonde.

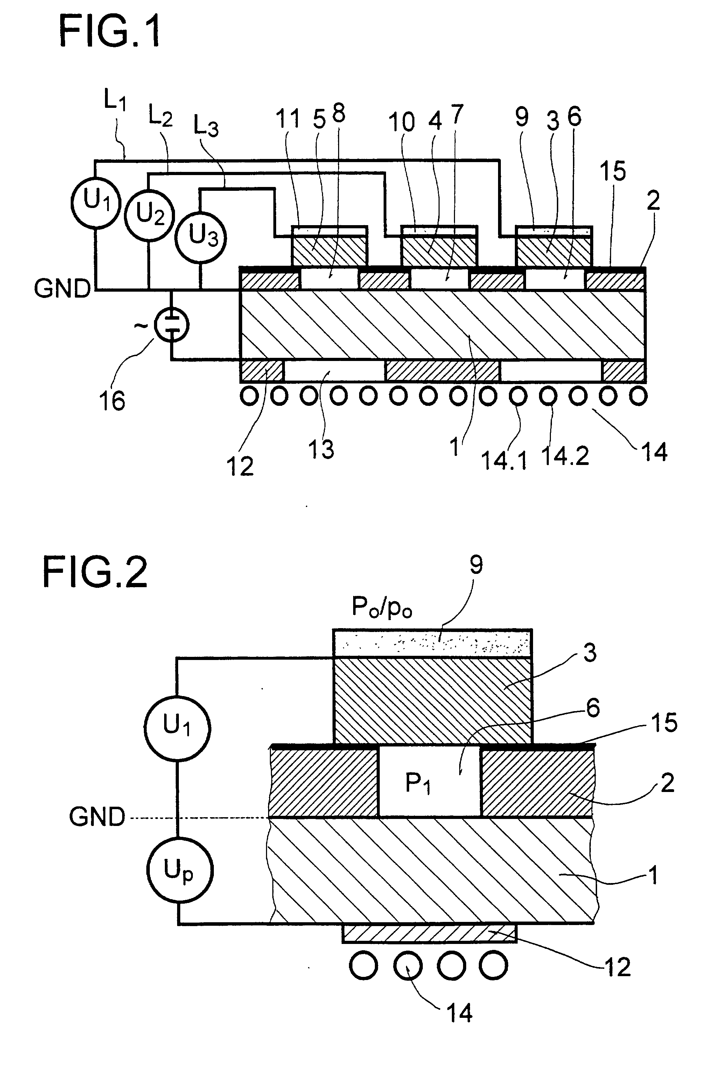

In Fig. 1 ist eine 3-Sensoren-Sonde gezeigt, die als Meßsonde zur Detektion der Momentan-Konzentration von Sauerstoff, $NH_3$ und NO geeignet ist. Ausgehend von einer Trägerplatte 1 aus einem sauerstoffleitenden Material, hier Zirkondioxid $ZrO_2$, ist auf einer Seite eine Schicht 2 aus elektrisch leitfähigem Material angeordnet. Die Auflage 2 ist mit Aussparungen versehen, die durch auf die Schicht 2 aufgesetzte Abdeckscheiben 3, 4, 5 Kammern 6, 7 ,8 bilden und die den ersten, zweiten und dritten Bereich der Meßsonde bilden.

Auf den Abdeckscheiben 3, 4, 5 sind Schichten 9, 10, 11 angeordnet, die aus drei unterschiedlichen, als Reaktionskatalysator wirkenden Materialien bestehen. Die Reaktionskatalysatoren sind zumindest teilselektiv, d.h. sie bevorzugen den Ablauf unterschiedlicher Reaktionen.

Auf der anderen Seite der Trägerplatte 1 ist eine weitere Schicht 12 aus einem elektrisch leitenden Material, beispielsweise aus demselben Material wie die Schicht 2, angeordnet, wobei hier dafür Sorge getragen werden muß, daß Sauerstoff in die Trägerplatte eindringen kann.

Aus diesem Grund ist diese Seite der Trägerplatte 1 nicht vollständig von der Schicht 12 bedeckt, vielmehr liegen Aussparungen 13 vor. Schließlich ist eine Heizung 14 vorgesehen, dargestellt durch die Heizdrähte 14.1, 14.2 usw.

Zur Erfassung der Spannungsdifferenz als Grundlage für die Bestimmung der Momentankonzentrationen sind Leiter vorgesehen, welche zwischen den Schichten 9, 10, 11 und den Scheiben 3, 4, 5 angeordnet sind. Der Grundleiter, GND, ist zwischen der Schicht 2 und der Trägerplatte 1 angeordnet.

Weiterhin ist eine Stromquelle 16 vorgesehen, welche einen Stromfluß zwischen der mit der Schicht 12 bedeckten Oberfläche der Trägerplatte 1 in die Kammern 6, 7, 8 hinein bzw. heraus ermöglicht. Als Folge des Stromflusses stellen sich in den Kammern 6, 7, 8 zeitlich veränderliche Sauerstoffpartialdrücke ein, die jedoch in den Kammern 6, 7, 8 untereinander gleich sind.

Die Stromquelle 16 wird so betrieben, daß in dem Sauerstoff messenden Bereich zwischen der Schicht 9 und dem Grundleiter 2 abwechselnd ein oberer und unterer Spannungsgrenzwert einer Spannung $U_1$ erreicht wird. Zur Bestimmung des $O_2$-Partialdrucks im Gasstrom wird die Zeitdifferenz zwischen dem Erreichen der Grenzwerte herangezogen. Diese Zeitdifferenz ist abhängig von der $O_2$-Konzentration im Abgas. Der $O_2$-Partialdruck in der Kammer 6 selbst ist unerheblich. Die Reaktionszeit der Meßsonde ist durch dieses Zeitintervall bestimmt und hängt von der Partialdruckdifferenz von $O_2$ in der Kammer 6 und in dem Gasstrom ab. Diese Differenz beträgt bei einer Reaktionszeit von 30 - 40 Millisekunden ca. 10 - 50 mbar.

In Fig. 2 ist eine Vergrößerung des ersten Bereichs der Meßsonde aus Fig. 1 dargestellt. Anhand dieser Vergrößerung wird die Funktionsweise der einzelnen Bereiche in Abhängigkeit des als Reaktionskatalysators wirkenden Materials der Beschichtung 9 erläutert. Ausgehend von einer bestimmten Sauerstoffkonzentration in der Kammer 6, bezeichnet als Partialdruck pi, stellt sich bei einem unterschiedlichen Sauerstoff-Partialdruck po als Maß für die im Gasstrom herrschende Konzentration bei einem Umgebungsdruck von Po die Spannungsdifferenz Ui ein.

Die Spannungsdifferenz Ui wird mittels eines nicht dargestellten Meßwertaufnehmers kontinuierlich oder in regelmäßigen Abständen erfaßt. Da kein Strom fließt, findet kein Stofftransport durch die Scheibe 3 aus beispielsweise Zirkondioxid statt.

Der Sauerstoff-Partialdruck $p_1$ in der Kammer 6 wird über die Schicht 12 als Pumpleiter innerhalb vorgegebener Grenzwerte einer Pumpspannung $U_p$ variiert, was durch die Ausnutzung eines unter Stromeinwirkung erzeugten Stofftransports von Sauerstoffionen durch die Trägerplatte aus Zirkondioxid erfolgt. Dabei findet an dem Pumpleiter durch die Verwendung eines entsprechenden Materials als Katalysator, vorzugsweise Pt, eine Aufspaltung von $O_2$-Molekülen in $O^-$-Ionen statt gemäß der folgenden Gleichung:

$$O_2 + 2\,e^- \rightleftharpoons 2\,O^-$$

Durch die Trägerplatte 1 hindurch wird die Kammer 6 mit Sauerstoffionen versorgt, wobei die Sauerstoffionen bei Erreichen der Grenzfläche zwischen Kammer und Trägerplatte 1 ihr Elektron wieder abgeben und als Sauerstoffmoleküle in die Kammer 6 eindringen.

Dadurch, daß alle Kammern 6, 7, 8 dieselbe Sauerstoffkonzentration, also denselben Sauerstoff-Partialdruck aufweisen, lassen sich unterschiedliche Spannungen $U_1$, $U_2$, $U_3$ (dargestellt in Fig. 1) für die einzelnen Bereiche dann messen, wenn die Beschichtungen 9, 10, 11 zu unterschiedlichen Sauerstoffkonzentrationen führten. Dies wird dadurch erreicht, daß die Beschichtung 9, 10, 11 aus unterschiedlichen Reaktionskatalysatoren bestehen. Im Ausführungsbeispiel ist der Reaktionskatalysator zur Messung des Sauerstoffgehalts Platin, welches unter Zuführung von Elektronen die Aufspaltung von $O_2$-Molekülen in Sauerstoffionen bewirkt. Zur Messung der $NH_3$-Konzentration wird als Reaktionskatalysator Silber (Ag) verwendet. Zur Messung der NO-Konzentration wird als Reaktionskatalysator Wolfram (W) verwendet.

An den drei unterschiedlichen Reaktionskatalysatoren in den ersten bis dritten Bereich finden demnach unterschiedliche, zumindest teilselektive Umsetzungen von NO und von $NH_3$ mit $O_2$ statt. Zusammen mit dem an dieser Stelle gemessenen Sauerstoffgehalt läßt sich aufgrund des Sauerstoffverbrauchs beim Ablaufen der Umsetzungsreaktionen anhand der stöchiometrischen Gleichungen auf die Momentan-Konzentration von NO bzw $NH_3$ schließen.

Aus der Spannungsdifferenz $U_1$-$U_2$ bzw. $U_1$-$U_3$ kann aufgrund der Reaktionsgleichungen auf den $O_2$-Verbrauch geschlossen werden und somit die Konzentration von NO und $NH_3$ im Gasstrom festgestellt werden. Der absolute $O_2$-Partialdruck im Abgasstrom ist hierfür ohne Bedeutung.

Zur gasdichten Verbindung der Plättchen 3, 4, 5 mit der Leiterschicht 2 wird ein auch bei 700°C nichtsauerstoffleitender Kleber 15 verwendet, mittels welchem ebenfalls die Verbindung der Plättchen 3, 4, 5 vorgestellt wird. Hierbei ist es wesentlich, daß der Kleber 15 selbst bei den Betriebstemperaturen der Meßsonde, die bei etwa 700°C liegt, nichtleitend für Sauerstoff bleibt. Als Kleber 15 eignet sich ein Glaspulver, welches nach der Auftragung in Pulverform auf die Trägerplatte 1 und der Plazierung der einzelnen Bauteile zueinander durch Erhitzen verflüssigt wird und anschließend beim Erstarren eine dichte Verbindung erzeugt. Der Kleber 15 kann sich als Schicht über die gesamte Oberfläche der Trägerplatte 1 erstrecken oder nur als Wulst im Bereich der Kammern angeordnet sein.

Mit der elektrischen Heizung wird die Sonde auf Temperaturen von 500 - 900°C, vorzugsweise 700°C, beheizt. Da die Sonde sehr klein ist, teilt sich diese hohe Temperatur der Gastemperatur praktisch nicht mit; selbst wenn dies der Fall wäre, hätte es keinen Einfluß auf das Verfahren.

Als Material für die Leiterschicht 2 und den Pumpleiter 12 eignet sich Platin. Zur Messung von $O_2$, $NH_3$ und NO wird die Zeit für den $O_2$-Transport durch die Trägerplatte hindurch in die Kammer 3, 4, 5 bis zu

einem gewünschten $O_2$-Partialdruck entsprechend einer bestimmten Nennspannung gemessen.

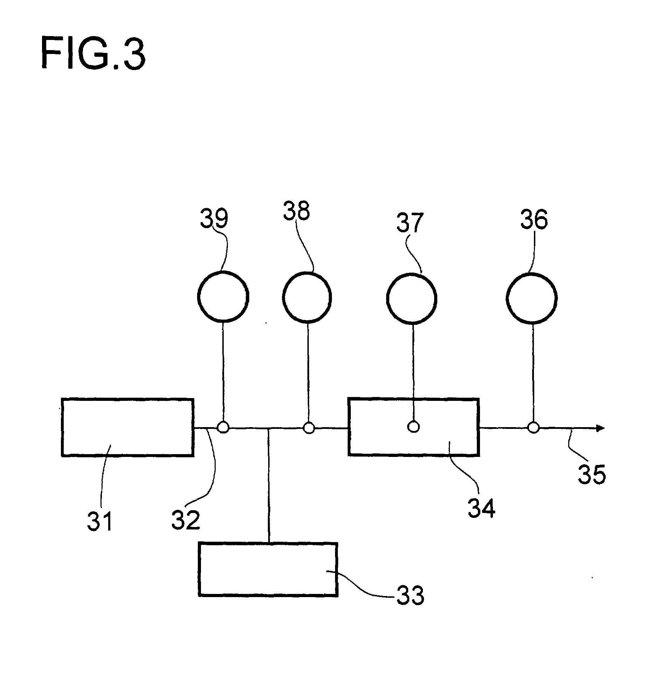

Die 3-Sensorensonde wird zur Bestimmung des momentanen Zustands des Abgases in einem DeNO$_x$-Katalysator-System gemäß Fig. 3 eingesetzt. Die Kenntnis des momentanen Zustands dient zu Regelung der Ammoniakzufuhr mit dem Ziel der Minimierung der Konzentrationen bzw. Mengen von $NH_3$ und NO nach Austritt aus dem Denox-Katalysator. Minimierung bedeutet in diesem Fall, daß der $NH_3$-Ausstoß und der NO-Ausstoß unter dem zulässigen Grenzwert liegen oder, als Spezialfall, NO unter dem zulässigen Grenzwert liegt und der $NH_3$-Ausstoß Null beträgt.

Zur Bestimmung des momentanen Zustands des Abgases des Katalysatorsystems genügen vier Sonden, wie sie in Fig. 3 eingezeichnet sind. Dem aus einer Verbrennungskraftmaschine 31 austretenden Abgas 32 wird durch Einspeisung von gasförmigem $NH_3$ oder durch Zufuhr ammoniakabgebender Substanzen aus einem Vorratsbehälter 33 $NH_3$ zugeführt. Das Abgas mit dem zugeführten $NH_3$ tritt in einen Katalysator 34 ein, in welchem die Stickoxide NO$_x$ mit dem $NH_3$ zumindest teilumgesetzt werden. Aus dem Katalysator 34 tritt dann der gereinigte Abgasstrom 35.

Die Sonde 36 nach dem Denox-Katalysator 34 gibt den erreichten Endzustand an. Die Sonde 37 im Katalysator 34, vorgezogen wird die Lage nach dem ersten Drittel der Katalysatorlänge, zeigt noch einen deutlich von Null verschiedenen Wert für $NH_3$ und NO an und gibt Auskunft über die Wirkung des Katalysators unter den gerade herrschenden Bedingungen von Ammoniakzufuhr, Temperatur, Katalysatoralter usw.

Die Sonde 38 unmittelbar vor dem Katalysator 34 zeigt die Zusammensetzung der Abgas- und Ammoniakmischung an. Die Sonde 39 unmittelbar nach der Verbrennungskraftmaschine 31 zeigt die Zusammensetzung des Abgases 32 an. An dieser Stelle enthält das Abgas noch kein Ammoniak. Wird hier durch die Sonde 39 Ammoniak angezeigt, so kann es sich nur um einen $NH_3$-vortäuschenden Bestandteil im Abgas handeln, der dann bei den nachfolgenden Sonden in Abzug gebracht werden muß.

Für die Erreichung des angestrebten Zieles scheinen folgende Sondenkombinationen möglich:

a) 36, 37, 38, 39
b) 36, 37
c) 36, 38
d) 37, 38
e) 36

Zusätzlich zu den Signalen der Sonden 36-39 kann zur Regelung auch noch die Motoreinstellung wie Drehzahl und Belastung sowie Gemischeinstellung herangezogen werden. Die geschilderten Messungen und Regelungen können auf alle Verbrennungsabgase angewendet werden, insbesondere auf die Verbrennungsabgase von Otto-Motoren und Diesel-Motoren für LKW und PKW, für Gasturbinen, Kohlekraftwerke, Müllheizkraftwerke, Blockheizkraftwerke, Sondermüllverbrennungsanlagen oder Tunnelabgase. Die Erfindung ist besonders vorteilhaft für schnell wechselnde Abgaszusammensetzungen, wie sie bei instationärer Fahr- und Betriebsweise auftreten.

Die Meßsonde wird zum Schutz gegen Verunreinigungen in einem Rohr untergebracht, welches zur Gasseite in bekannter Weise mittels einer Sintermetallplatte gegen das Eindringen von Feststoffpartikeln geschützt ist. Da die Meßsonde auf eine Temperatur oberhalb des Versottungspunktes beheizt ist, bilden sich an der Meßsonde keine Kondensate aus. Wegen der hohen Temperatur der Sonde werden etwaige übrige Rückstände verbrannt, sodaß eine hohe Funktionssicherheit gewährleistet ist.

Die 3-Sensoren-Sonde besteht aus drei Zirkondioxidzellen ($ZrO_2$-Zellen), die auf bekannte Weise bei erhöhter Temperatur von 500-900°C als Festelektrolyt Sauerstoff in Form von Ionen transportieren können.

Der Ammoniaksensor in der 3-Sensoren-Sonde funktioniert folgendermaßen: Auf die Scheibe 4 aus Zirkondioxid ist eine dünne Schicht eines katalytischen Materials aufgebracht, in dem bei 500-900°C Ammoniak mit Sauerstoff umgesetzt wird. Dieser Reaktionskatalysator ist zum Beispiel Silber. Die Umsetzung in diesem Bereich findet nach der folgenden Gleichung statt:

$$4\ NH_3 + 3\ O_2 \rightarrow 2\ N_2 + 6H_2O\ \text{(mit Ag)}$$

Aus der dadurch verminderten Sauerstoffkonzentration, gemessen im Vergleich zum unmittelbar benachbarten Sauerstoffsensor, ergibt sich die zugrundeliegende Ammoniakkonzentration

Der Sensor innerhalb der 3-Sensoren-Sonden für Stickstoffoxid enthält auf der Scheibe 5 aus Zirkondioxid eine dünne Schicht eines katalytisch wirksamen Materials, beispielsweise Wolfram, an dem die Umsetzung von Stickstoffoxid mit Ammoniak und Sauerstoff erfolgt. Dies geschieht nach der folgenden Reaktionsgleichung:

$$4\ NO + 4\ NH_3 + O_2 \rightarrow 4\ N_2 + 6\ H_2O\ \text{(mit Katalysator)}$$

Der Verbrauch an Sauerstoff an diesem Reaktionskatalysator, zum Beispiel Wolfram (W), gegenüber dem Meßergebnis des unmittelbar benachbarten Sauerstoffsensors, ist ein Maß für die zugrundeliegende Stickstoffoxidkonzentration.

Die beiden genannten Reaktionen brauchen an den Reaktionskatalysatoren, von denen hier beispielsweise Ag und W genannt sind, nicht selektiv und nicht 100%ig abzulaufen. Die jeweilige Reaktion muß lediglich zu einem deutlich größeren Anteil ablaufen. Am $NH_3$-Reaktionskatalysator Silber kann auch die andere Reaktion, nämlich die von NO, NH$_3$ und O$_2$ teilweise erfolgen. Ferner kann die hier zugeordnete NH$_3$-Oxidation auch zu etwas weniger als 100% erfolgen. Ebenso läuft die Reaktion von NO, NH$_3$ und O$_2$ am Wolfram lediglich überwiegend ab. Durch Eichung lassen sich die Sauerstoffverbräuche, gemessen als Defizit gegenüber dem Meßergebnis des Sauerstoffsensors, den Konzentrationen von NH$_3$ und NO zuordnen.

Die Reaktionskatalysatoren können sein für den NH$_3$-Sensor: Ag, Pd, Pt, Ru, Ir, In, Ni, TiO$_2$, für den NO-Sensor W, V205. Die genannten Reaktionskatalysatoren sind in dünner Schicht auf das Zirkondioxid aufgebracht. Sie können im Verlaufe der Reaktion in umgewandelter Form vorliegen, etwa als Oxid oder mit adsorbiertem Sauerstoff.

Durch die Zusammenfassung der drei Sensoren zu einer kleindimensionierten 3-Sensoren-Sonde mit Abmessungen von ungefähr 1x1x1 cm werden zur gleichen Zeit am gleichen Punkt die Gaskonzentrationen von Sauerstoff, NH$_3$ und NO gemessen. Falls in einem Katalysatorsystem mehrere Sonden angeordnet sind, weiß man über den Konzentrationsverlauf und den Reaktionsverlauf der Abgaskomponenten O$_2$, NH$_3$ und NO entlang des Abgasstroms vom Motorausgang bis zum Katalysatorende Bescheid. Die Meßgeschwindigkeit ist um so größer, je kleiner das Volumen der Kammern 3, 4, 5 ist und liegt im Bereich von Millisekunden bis Zehntelsekunden. Dadurch kann auch die zeitliche Veränderung der Abgaskomponenten selbst bei extrem instationärem Betrieb gemessen werden. Die Regelung der Ammoniakzufuhr kann dadurch unmittelbar erfolgen. Aufgrund der Zusammenfassung der drei Sensoren auf einer einzigen Meßsonde werden neue Möglichkeiten eröffnet, wie sie nach dem bisherigen Stand der Technik nicht möglich erschienen.

Mit der erfindungsgemäßen Meßsonde lassen sich folgende Probleme lösen:

Da die Meßgeschwindigkeit infolge der Kleinheit sehr groß ist, können die Signale im Millisekunden- bis Zehntelsekundenbereich erzeugt und verwertet werden. Schnelle Änderungen bei instationärer Betriebsweise werden sofort erfaßt.

Da die drei Gaskomponenten O$_2$, NH$_3$ und NO gleichzeitig und nahezu am selben Ort gemessen werden, sind die wichtigsten Reaktanten des Abgases gleichzeitig bekannt.

Da die Bezugsgröße O$_2$ immer am gleichen Ort wie NH$_3$ und NO mitgemessen wird, tritt auch bei rascher Änderung der O$_2$-Konzentration im Abgas keine Falschmeldung auf.

Da die Meßsonde klein und kompakt ist, kann sie an vielen Stellen des Abgaswegs zwischen dem Austritt des Abgases aus der Verbrennungsmaschine und dem Austritt des gereinigten Abgases nach dem Katalysator eingesetzt werden. Dadurch ist auch die Abgasänderung längs des Abgaswegs erfaßbar. Vorteilhaft wären vier Meßstellen.

Die bei vier Meßstellen insgesamt 12 Signale, je 4 für O$_2$, NH$_3$ und NO, können mittels Fuzzy-Logic zur Regelung der NH$_3$-Zufuhr und damit zur Minimierung der NH$_3$- und NO-Menge am Austritt verwertet werden.

Zur Verbesserung der Meßgenauigkeit des Gesamtsystems kann vor der NH$_3$-Einspeisung die Meßsonde 39 zur Erkennung von beispielsweise durch Kohlenwasserstoffe vorgetäuschtem NH$_3$-Signal erfolgen und bei den nachfolgenden Sensoren berücksichtigt werden. Die dazu erforderliche Sonde könnte als Vergleichssonde bezeichnet werden.

## Patentansprüche

1. Meßsonde für die Detektion der Momentankonzentrationen mehrerer Gasbestandteile eines Gases, **dadurch gekennzeichnet, daß** ein erster Bereich vorhanden ist, in dem die Momentankonzentration von Sauerstoff erfaßt wird, daß ein zweiter Bereich vorhanden ist, in dem die Momentankonzentration von NH$_3$ bestimmt wird und der einen Reaktionskatalysator zumindest zur teilselektiven Umsetzung von NH$_3$ mit O$_2$ aufweist, und daß ein dritter Bereich vorhanden ist, in dem die Momentankonzentration von NO bestimmt wird und der einen Reaktionskatalysator zumindest zur teilselektiven Umsetzung von NO und NH$_3$ mit O$_2$ aufweist.

2. Meßsonde nach Anspruch 1, **dadurch gekennzeichnet, daß** ein vierter Bereich zur Bestimmung der Momentankonzentration von CO vorhanden ist.

3. Meßsonde nach Anspruch 1, daß der erste Bereich zur Erfassung des Partialdrucks von O$_2$ ausgebildet ist.

4. Meßsonde nach einem der Ansprüche 1 bis 3, **dadurch gekennzeichnet, daß** die Bereiche auf einem gemeinsamen Träger (1) angeordnet sind.

5. Meßsonde nach einem der Ansprüche 1 bis 4, **dadurch gekennzeichnet, daß** eine Heizvorrichtung (14) vorgesehen ist.

6. Meßsonde nach Anspruch 5, **dadurch gekennzeichnet, daß** die Heizvorrichtung (14) derart dimensioniert ist, daß eine Temperatur von 500 bis 900°C erreicht wird.

7. Meßsonde nach einem der Ansprüche 1 bis 6, **dadurch gekennzeichnet, daß** eine Referenzschicht (12) aus einem Referenzmaterial mit dem Träger (1) verbunden ist, wobei der Träger (1) aus einem Material besteht, in welchem O$_2$ in Form von Ionen transportiert werden kann, daß auf der anderen Seite des Trägers (1) eine Schicht (2) aus dem Referenzmaterial mit Kammern (6, 7, 8) angeordnet ist, daß die Kammern (6, 7, 8) durch Scheiben (3, 4, 5) aus einem Material, in welchem $O_2$ in Form von Ionen transportiert werden kann, abgedeckt und gasdicht verschlossen sind und daß auf den Scheiben (3, 4, 5) die Reaktionskatalysatoren (9, 10, 11) angeordnet sind.

**8.** Meßsonde nach Anspruch 7, **dadurch gekennzeichnet, daß** Mittel zur zeitlichen Erfassung der Spannung zwischen den Reaktionskatalysatoren (9, 10, 11) und der Referenzschicht vorgesehen sind.

**9.** Meßsonde nach einem der Ansprüche 7 oder 8, **dadurch gekennzeichnet, daß** zwischen den Scheiben (3, 4, 5) und dem Träger (1) ein Glaskleber (15) angeordnet ist.

**10.** Katalysatorsystem zur Reduktion von Stickoxiden in Verbrennungsgasen durch Umsetzung mit $NH_3$, **dadurch gekennzeichnet, daß** die Meßsonde (36, 37) gemäß einem der Ansprüche 1 bis 9 zumindest am Katalysatorausgang und/oder im Katalysator nach der Stelle der $NH_3$-Einspeisung angeordnet ist.

**11.** Katalysatorsystem nach Anspruch 10, **dadurch gekennzeichnet, daß** die Meßsonde (38, 39) zusätzlich noch unmittelbar vor dem Katalysator und/oder vor einer Stelle der $NH_3$-Einspeisung angebracht ist.

**12.** Verfahren zur Minimierung des $NH_3$- und NO-Ausstoßes aus einem Katalysatorsystem gemäß einem der Ansprüche 10 bis 12, **dadurch gekennzeichnet, daß** man die Meßergebnisse der Meßsonden zur Regelung der $NH_3$-Einspeisung verwendet.

## Claims

**1.** A sensor for detecting the instantaneous concentrations of a plurality of gas constituents of a gas, **characterized in that** there is a first zone in which the instantaneous concentration of oxygen is measured, **in that** there is a second zone in which the instantaneous concentration of $NH_3$ is measured and which contains a reaction catalyst at least for partially selective reaction of $NH_3$ with $O_2$, and **in that** there is a third zone in which the instantaneous concentration of NO is measured and which contains a reaction catalyst at least for partially selective reaction of NO and $NH_3$ with $O_2$.

**2.** A sensor as claimed in claim 1, **characterized in that** a fourth zone is present for measuring the instantaneous concentration of CO.

**3.** A sensor as claimed in claim 1, **characterized in that** the first zone is designed to measure the partial pressure of $O_2$.

**4.** A sensor as claimed in any one of claims 1 to 3, **characterized in that** the zones are arranged on a common substrate (1).

**5.** A sensor as claimed in any one of claims 1 to 4, **characterized in that** a heating device (14) is provided.

**6.** A sensor as claimed in claim 5, **characterized in that** the heating device (14) is of such dimensions that a temperature of from 500 to 900°C is achieved.

**7.** A sensor as claimed in any one of claims 1 to 6, **characterized in that** a reference layer (12) of a reference material is bonded to the substrate (1), the substrate (1) being of a material in which $O_2$ can be transported in the form of ions, **in that** a layer (2) of the reference material with chambers (6, 7, 8) is arranged on the other side of the substrate (1), **in that** the chambers (6, 7, 8) are covered and sealed in a gastight manner by sheets (3, 4, 5) of a material in which $O_2$ can be transported in the form of ions, and **in that** the reaction catalysts (9, 10, 11) are arranged on the sheets (3, 4, 5).

**8.** A sensor as claimed in claim 7, **characterized in that** means for recording the voltage between the reaction catalysts (9, 10, 11) and the reference layer over time are provided.

**9.** A sensor as claimed in claim 7 or claim 8, **characterized in that** a glass adhesive (15) is arranged between the sheets (3, 4, 5) and the substrate (1).

**10.** A catalyst system for reducing nitrogen oxides in combustion gases by reaction with $NH_3$, **characterized in that** the sensor (36, 37) as claimed in any one of claims 1 to 9 is arranged at least at the catalyst outlet and/or in the catalyst after the $NH_3$ feedpoint.

**11.** A catalyst system as claimed in claim 10, **characterized in that** the sensor (38, 39) is additionally installed immediately before the catalyst and/or before an $NH_3$ feedpoint.

**12.** A process for minimizing the output of $NH_3$ and NO from a catalyst system as claimed in any one of claims 10 to 11, **characterized in that** the measurement results from the sensors are used to regulate the $NH_3$ feed.

**Revendications**

1. Sonde de mesure pour la détection des concentrations instantanées de plusieurs composants gazeux d'un gaz, **caractérisée en ce qu'**il existe une première zone dans laquelle la concentration instantanée en oxygène est déterminée, **en ce qu'**il existe une deuxième zone dans laquelle la concentration instantanée en $NH_3$ est déterminée et qui présente un catalyseur de réaction au moins pour la réaction partiellement sélective de $NH_3$ avec $O_2$, et **en ce qu'**il existe une troisième zone dans laquelle la concentration instantanée en NO est déterminée et qui présente un catalyseur de réaction au moins pour la réaction partiellement sélective de NO et $NH_3$ avec $O_2$.

2. Sonde de mesure selon la revendication 1, **caractérisée en ce qu'**il existe une quatrième zone pour la détermination de la concentration instantanée en CO.

3. Sonde de mesure selon la revendication 1, **caractérisée en ce que** la première zone est conçue pour la détermination de la pression partielle d'$O_2$.

4. Sonde de mesure selon l'une quelconque des revendications 1 à 3, **caractérisée en ce que** les zones sont disposées sur un support commun (1).

5. Sonde de mesure selon l'une quelconque des revendications 1 à 4, **caractérisée en ce qu'**un dispositif de chauffage (14) est prévu.

6. Sonde de mesure selon la revendication 5, **caractérisée en ce que** le dispositif de chauffage (14) est dimensionné de manière à pouvoir atteindre une température de 500°C à 900°C.

7. Sonde de mesure selon l'une quelconque des revendications 1 à 6, **caractérisée en ce qu'**une couche de référence (12) composée d'une matière de référence est liée au support (1), le support (1) étant composé d'une matière dans laquelle $O_2$ peut être transporté sous forme d'ions, **en ce qu'**une couche (2) composée de la matière de référence comprenant des compartiments (6, 7, 8) est disposée de l'autre côté du support (1), **en ce que** les compartiments (6, 7, 8) sont recouverts et rendus étanches aux gaz par des plateaux (3, 4, 5) composés d'une matière dans laquelle $O_2$ peut être transporté sous forme d'ions, et **en ce que** des catalyseurs de réaction (9, 10, 11) sont disposés sur les plateaux (3, 4, 5).

8. Sonde de mesure selon la revendication 7, **caractérisée en ce que** des moyens pour la détermination temporelle de la tension entre les catalyseurs de réaction (9, 10, 11) et la couche de référence sont prévus.

9. Sonde de mesure selon l'une quelconque des revendications 7 ou 8, **caractérisée en ce qu'**une colle pour verre (15) est disposée entre les plateaux (3, 4, 5) et le support (1).

10. Système de catalyseur pour la réduction des oxydes d'azote dans les gaz de combustion par la réaction avec $NH_3$, **caractérisé en ce que** la sonde de mesure (36, 37) selon l'une quelconque des revendications 1 à 9 est disposée au moins à la sortie du catalyseur et/ou dans le catalyseur en aval de la position de l'arrivée de $NH_3$.

11. Système de catalyseur selon la revendication 10, **caractérisé en ce que** la sonde de mesure (38, 39) est disposée en plus immédiatement devant le catalyseur et/ou devant une position de l'arrivée de $NH_3$.

12. Procédé pour minimiser les rejets de $NH_3$ et de NO à partir d'un système de catalyseur selon l'une quelconque des revendications 10 à 12, **caractérisé en ce que** l'on utilise les résultats de mesure des sondes de mesure pour réguler l'arrivée de $NH_3$.

# FIG.1

# FIG.2

# FIG.3